(19) Europäisches Patentamt
European Patent Office
Office européen des brevets

(11) Veröffentlichungsnummer: **0 196 573 A2**

(12) # EUROPÄISCHE PATENTANMELDUNG

(21) Anmeldenummer: **86103878.4**

(22) Anmeldetag: **21.03.86**

(51) Int. Cl.[4]: **C07C 113/04**, C09B 35/08, D06P 3/68

(30) Priorität: **29.03.85 DE 3511547**

(43) Veröffentlichungstag der Anmeldung: **08.10.86 Patentblatt 86/41**

(84) Benannte Vertragsstaaten: **BE CH DE FR GB IT LI NL**

(71) Anmelder: **HOECHST AKTIENGESELLSCHAFT**
**Postfach 80 03 20**
**D-6230 Frankfurt am Main 80(DE)**

(72) Erfinder: **Hertel, Hasso, Dr.**
**Brunnenweg 10**
**D-6052 Mühlheim am Main(DE)**
Erfinder: **Hunger, Klaus, Dr.**
**Johann-Strauss-Strasse 35**
**D-6233 Kelkheim (Taunus)(DE)**
Erfinder: **Frölich, Heinrich, Dr.**
**Oberhäuser Weg 6**
**D-6272 Niedernhausen/Taunus)(DE)**

(54) **bis-Diazoniumsalze von 4,4'-Diamino-3,3'-dialkoxy-biphenylen, Verfahren zu ihrer Herstellung und deren Verwendung.**

(57) bis-Diazoniumsalze der Formel (1)

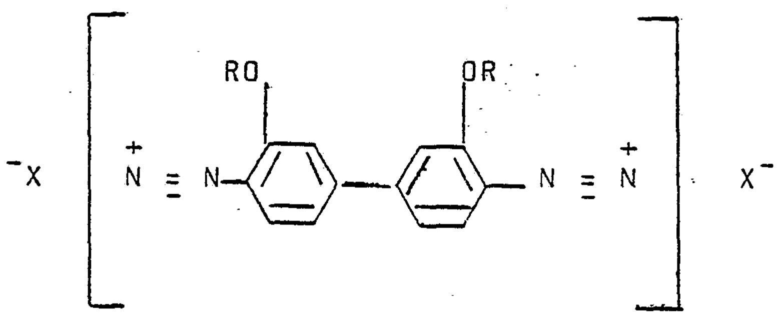

(1)

worin R einen geradkettigen oder verzweigten Alkyl-oder Alkoxyalkylrest von 3-5 Kohlenstoffatomen bedeutet, und $X^-$ den Rest einer starken Säure oder eines Zinkchloriddoppelsalzes darstellt, und Verfahren zu ihrer Herstellung, indem man ein 4,4'-Diamino-3,3'-dialkoxy-biphenyl der Formel (2)

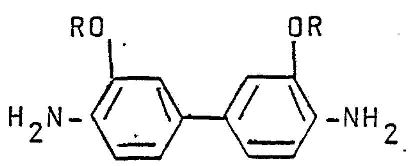

(2)

worin R die genannte Bedeutung hat, in einer wäßrigen, starken, nichtoxydierenden anorganischen oder organischen Säure bei Temperaturen von etwa -10°C bis etwa +40°C mit einem Alkalimetallnitrit diazotiert und die gebildeten bis-Diazoniumsalze durch Zugeben eines Alkalimetallchlorids, -hydrogensulfats, -monosulfonats der 1,5-Naphthalindisulfonsäure, -tetrafluoborats oder von Zinchlorid abscheidet.

EP 0 196 573 A2

bis-Diazoniumsalze von 4,4'-Diamino-3,3'-dialkoxy-biphenylen, Verfahren zu ihrer Herstellung und deren Verwendung

Die vorliegende Erfindung betrifft neue bis-Diazoniumsalze von 4,4'-Diamino-3,3'-dialkoxy-biphenylen, Verfahren zu ihrer Herstellung und deren Verwendung zum Färben oder Bedrucken von Textilfasermaterialien nach den Methoden der Eisfarbentechnik, d.h. zur Erzeugung von wasserunlöslichen Azofarbstoffen, insbesondere auf der Faser, unter Verwendung geeigneter Kupplungskomponenten, sowie zur Herstellung von Lichtpausen nach den Methoden der Reprografie.

Gefunden wurden neue bis-Diazoniumsalze der allgemeinen Formel (1)

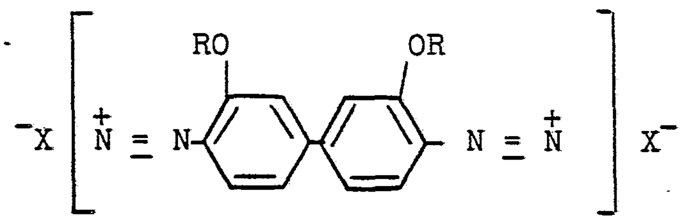
(1)

in welcher R einen geradkettigen oder verzweigten Alkyl- oder Alkoxyalkylrest von insgesamt 3 bis 5 Kohlenstoffatomen bedeutet, und $X^-$ den Rest einer starken Säure oder eines Zinkchloriddoppelsalzes darstellt, Verfahren zu ihrer Herstellung, in dem man ein 4,4'-Diamino-3,3'-dialkoxy-biphenyl der Formel (2)

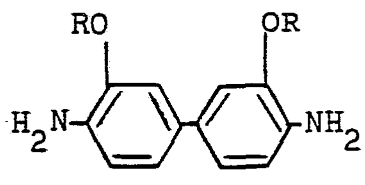
(2)

in welcher R die vorstehend genannten Bedeutungen hat, in einer wäßrigen, starken, nichtoxydierenden anorganischen oder organischen Säure, wie wäßriger Schwefelsäure, Phosphorsäure, Chloressigsäure oder vorzugsweise Salzsäure, mit einem Alkalimetallnitrit, vorzugsweise Natriumnitrit, bei Temperaturen von etwa -10°C bis etwa +40°C, vorzugsweise 0 bis 30°C, bis-diazotiert und die gebildeten bis-Diazoniumsalze durch Zugabe eines Alkalimetallchlorids, -hydrogensulfats, -monosulfonats der 1,5-Naphthalindisulfonsäure, -tetrafluorborats, beispielsweise der Kalium-oder vorzugsweise der Natriumsalze, oder von Zinkchlorid abscheidet.

Demgemäß bedeutet der Anionrest $X^-$ in der genannten allgemeinen Formel (1)

$Cl^-$, $HSO_3^-$, 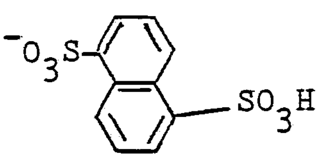 ,

$BF_4^-$ oder $\left[\frac{ZnCl_4}{2}\right]^-$ .

Die Abscheidung der bis-Diazoniumsalze kann durch Aussalzen mit Alkalimetallchloriden, wie Natrium-oder Kaliumchlorid, oder durch Einengen der angefallenen Lösungen bzw. Suspensionen vervollständigt werden.

Die wäßrige, starke Säure, in welcher die Bis-diazotierung vorgenommen wird, hat zweckmäßigerweise eine Konzentration von etwa 3 % bis etwa 18 %. Die im Einzelfall anzuwendende optimale Konzentration richtet sich nach der eingesetzten Säure.

Es ist zweckmäßig, pro Mol Diamin 4,4 bis 10 Äquivalente, vorzugsweise 5 bis 6 Äquivalente an Säure anzuwenden.

Als Diamine der genannten Formel (2) kommen in Betracht 4,4'-Diamino-3,3'-dipropoxy-biphenyl, 4,4'-Diamino-3,3'-bis-(1-methyl-ethoxy)-biphenyl, 4,4'-Diamino-3,3'-dibutoxy-biphenyl, 4,4'-Diamino-3,3'-bis-(1-methyl-propoxy)-biphenyl, 4,4'-Diamino-3,3'-bis-(2-methyl-propoxy)-biphenyl, 4,4'-Diamino-3,3'-bis-(1,1-dimethyl-ethoxy)-biphenyl, 4,4'-Diamino-3,3'-dipentoxy-biphenyl, 4,4'-Diamino-3,3'-bis-(3-methyl-butoxy)-biphenyl, 4,4'-Diamino-3,3'-bis-(2-methoxy-ethoxy)-biphenyl und 4,4'-Diamino-3,3'-bis-(2-ethoxy-ethoxy)-biphenyl.

Die Herstellung der genannten Diamine kann beispielsweise durch Reduktion von o-Nitro-alkoxy-benzolen in alkalischem Medium zu Diarylhydrazinen, die in stark saurem Medium zu den 4,4'-Diamino-3,3'-Dialkoxy-biphenylen umgelagert werden, erfolgen.

Damit die neuen bis-Diazoniumsalze der genannten allgemeinen Formel (1) in vorteilhafter Weise zusammen mit geeigneten Kupplungskomponenten zur Herstellung wasserunlöslicher Azofarbstoffe auf Textilfasern, vorzugsweise solchen aus nativer oder regenerierter Cellulose, eingesetzt werden können, ist es zweckmäßig, die Diazoniumsalze in hierfür geeignete Präparate überzuführen. Dies kann beispielsweise dadurch erfolgen, daß man das von der Herstellung her noch feuchte Diazoniumsalz, wie beispielsweise das Tetrachlorozinkat, mit der etwa gleichen Gewichtsmenge Natriumsulfat vermischt und dann im Luftstrom bei 40°C trocknet, oder in dem man das von der Herstellung her noch feuchte Diazoniumsalz mit Aluminiumsulfathexahydrat, Magnesiumsulfatmonohydrat und Natriumsulfat vermischt, wobei ggfs. auftretende Hydratationswärme durch Außenkühlung abgeführt wird. Die so hergestellten Mischungen werden zweckmäßigerweise schließlich durch Zugabe von weiterem Natriumsulfat auf einen bestimmten Prozentgehalt, beispielsweise von etwa 20 %, an Diazoniumsalz eingestellt.

Die Applikation der inredestehenden bis-Diazoniumsalze in Form der erwähnten Präparate zusammen mit geeigneten Kupplungskomponenten (Eisfarbentechnik) ist im Prinzip bekannt und beispielsweise in K. Venkataraman, The Chemistry of Synthetic Dyes, Volume I, Seiten 650-740, insbesondere Seiten 668-672, Academic Press, New York, 1952, beschrieben. Die Verwendung der bis-Diazoniumsalze zur Herstellung von Lichtpausen nach den Methoden der Reporgrafie wird im Prinzip beispielsweise in Kirk-Othmer, Encyclopedia of Chemical Technology, Second Edition, Volume 17, Seiten 358-364, Interscience Publishers, New York, 1968, beschrieben.

Beispiel 1

328 Teile 4,4'-Diamino-3,3'-dibutoxy-biphenyl werden in 600 Teil Wasser eingetragen und darin gut verrührt. Dann werden 260 Teile 32 %iger Salzsäure zugetropft, wobei sich ein Brei des Hydrochlorids bildet. Nach einstündigem Nachrühren zur Vervollständigung der Hydrochloridbildung werden weitere 320 Teile 32 %iger Salzsäure zugegeben. Durch Außenkühlung wird auf ca. 20°C abgekühlt. Dann läßt man 370 Teile einer 40 %igen wäßrigen Lösung von Natriumnitrit so rasch zutropfen, daß Nitrit zunächst nicht oder nur schwach im Überschuß ist. Die Temperatur steigt dabei auf 30-35°C an und wird auf diesem Niveau bis zum Ende der Diazotierung gehalten. Nach beendeter Diazotierung soll ein deutlicher Nitritüberschuß vorhanden sein.

Anschließend gibt man 10 Teile Klärkohle zu. Nach etwa 5 Minuten wird durch Tüpfeln der Auslauf geprüft. Ist er rein gelb, so wird nach Zugabe von 10 Teilen Kieselgur abgesaugt, ist er noch braun, so muß erneut Kohle zugegeben werden. Der Rückstand wird mit wenig Wasser gewaschen.

Bereits während der Klärung wird die Temperatur des Ansatzes abgesenkt, was erforderlichenfalls bei der nun folgenden Fällung fortgesetzt wird, bis ca. 15°C erreicht sind.

Zur Fällung des bis-Diazoniumsalzes läßt man zu der leicht bräunlichen Diazoniumsalzlösung 450 Teile wäßrige 33 %ige Zinkchloridlösung zutropfen. Hierbei scheidet sich das 3,3'-Dibutoxy-biphenyl-4,4'-bis(diazonium)-tetrachlorozinkat in schönen gelben Kristallen ab. Durch Zugabe von 250 Teilen Natriumchlorid wird die Fällung vervollständigt. Dann wird das bis-Diazoniumsalz abgesaugt und durch Zentrifugieren vom größten Teil der anhaftenden Mutterlauge befreit. Der Restfeuchtegehalt beträgt noch ca. 8 %.

Beispiel 2

a) Zur Herstellung eines Präparats zur Erzeugung von wasserunlöslichen Azofarbstoffen auf der Faser wird das nach Beispiel 1 erhaltene feuchte bis-Diazoniumsalz - (Tetrachlorozinkat) in einem säurefest ausgekleideten Mischer, der einen Kühlmantel besitzt, mit 50 Teilen Aluminiumsulfathexahydrat, 130 Teilen Magnesiumsulfatmonohydrat und 380 Teilen Natriumsulfat vermischt. Man geht dabei so vor, daß man die anorganischen Salze vorlegt und das feuchte bis-Diazoniumsalz portionsweise einträgt. Die dabei auftretende Hydratationswärme wird durch Außenkühlung mit kaltem Wasser abgeführt.

Nach einer Bestimmung des Reingehalts wird dieser durch Zugabe von weiterem Natriumsulfat auf einen bestimmten Wert, z.B. 20 %, bezogen auf Mol 328, eingestellt.

b) Herstellung einer Färbung auf einem Fasermaterial aus nativer Cellulose:

2,8 Teile 2-Hydroxynaphthalin-3-carbonsäure-(naphthyl-1)-amid werden mit einer Mischung aus 4 Teilen Äthanol, 4 Teilen Wasser, 1,4 Teilen 32 %iger Natronlauge und 1,4 Teilen 33 %igem Formaldehyd übergossen und durch Rühren gelöst. Nach zehn Minuten wird diese Lösung in 1000 Teile Wasser, die 8,5 Teile 32 %ige Natronlauge und 2 Teile eines Fettsäure-Eiweißabbauprodukt-Kondensates enthalten, eingegossen. In dieses Bad werden 50 Teile gebleichtes, abgekochtes und vorgenetztes Baumwollgarn eingebracht und darin unter gutem Umziehen 30 Minuten behandelt. Anschliessend wird das Färbegut herausgenommen, in einer Lösung von 30 Teilen Natriumchlorid und 1,3 Teilen 32 %iger Natronlauge in 1000 Teilen Wasser gespült und in ein Entwicklungsbad gebracht, das wie folgt bereitet wird:

6,75 Teile des vorstehend genannten Färbepräparats sowie 5 Teile Chromacetat grün und 1 Teil eines Alkylpolyglykolethers werden in 1000 Teilen Wasser gelöst. In diesem Entwicklungsbad wird das eingebrachte Textilmaterial 30 Minuten behandelt, sodann herausgenommen, wie üblich gespült, geseift und getrocknet.

Es wird eine blaue Färbung in guter Farbausbeute und guten Echtheiten erhalten.

Beispiel 3

a) Zur Hezstellung eines Präparats zur wasserunlöslichen Azofarbstoffen auf der Faser, welches frei von wasserunlösliche Hydoxide bildenden Metallsalzen ist, wird das nach Beispiel 1 erhaltene feuchte bis-Diazoniumsalz mit der gleichen Menge Natriumsulfat vermischt und sodann im Warmluftstrom von 40°C getrocknet.

Nach einer Bestimmung des Reinheitsgehalts wird dieser

durch Zugabe von weiterem Natriumsulfat auf einen bestimmten Wert, z.B. 20 %, bezogen auf Mol 328, eingestellt.

b) Herstellung einer Färbung auf Baumwollgarn:

8 Teile 2-Hydroxynaphthalin-3-carbonsäure-(4-chlor-2-methyl-phenyl)-amid werden mit 13 Teilen Äthanol angeteigt und durch Zugabe von 5,5 Teilen 32 %iger Natronlauge sowie 16 Teilen Wasser von 40°C gelöst. Nun werden noch 4 Teile 33 %ige Formaldehydlösung eingetragen. Nach 10 Minuten wird diese Stammlösung in eine Lösung von 44 Teilen 32 %iger Natronlauge, 120 Teilen Natriumchlorid und 18 Teilen eines handelsüblichen Eiweißabbauprodukt-Fettsäure-Kondensats in 6000 Teilen weichem Wasser von 35°C gegeben. Mit dieser Flotte wird in einem kleinen Kreuzspulapparat eine Kreuzspule mit 600 Teilen Baumwollgarn, die zur Entfernung von störenden Nichtcellulose-Substanzen alkalisch mit einem Tensid und Sequestriermittel abgekocht worden war, 30 Minuten lang behandelt.

Nach Ablassen der Flotte wird die grundierte Spule 10 Minuten lang mit einer Lösung von 240 Teilen Natriumchlorid und 4 Teilen 32 %iger Natronlauge in 6000 Teilen Wasser gespült. Wenn das Spülbad abgelassen ist, wird durch die Kreuzspule eine Entwicklungsflotte von ca. 20°C gepumpt, die wie folgt bereitet wurde:

In 6000 Teilen Wasser werden 30 Teile des gemäß Absatz a) hergestellten Färbepräparats sowie 22 Teile Natriumdihydrogenphosphat-dihydrat, 48 Teile Dinatriumhydrogenphosphat-dodekahydrat und 12 Teile eines Octadecylalkoholpolyglykolethers gelöst. Nach etwa 30 Minuten wird die Entwicklungsflotte abgelassen, die Kreuzspule wie üblich sauer gespült, kalt klargespült, erst bei 60°C, dann bei 100°C geseift, anschließend warm und kalt gespült und schließlich getrocknet.

Man erhält eine Blaufärbung in guter Farbausbeute und guten Echtheiten.

Beispiel 4

a) 300 Teile 4,4'-Diamino-3,3'-dipropoxy-biphenyl werden in 600 Teile Wasser eingetragen und darin gut verrührt. Dann werden 260 Teile 32 %iger Salzsäure zugetropft, wobei sich ein Brei des Hydrochlorids bildet. Nach einstündigem Nachrühren zur Vervollständigung der Hydrochloridbildung werden weitere 320 Teile 32 %iger Salzsäure zugegeben. Durch Außenkühlung wird auf ca. 20°C abgekühlt. Dann läßt man 370 Teile einer 40 %igen wäßrigen Lösung von Natriumnitrit so rasch zutropfen, daß Nitrit zunächst nicht oder nur schwach im Überschuß ist. Während der Diazotierung wird die Temperatur bei 10-15°C gehalten. Nach beendeter Diazotierung soll ein deutlicher Nitritüberschuß vorhanden sein.

Anschließend gibt man 10 Teile Klärkohle zu. Nach etwa 5 Minuten wird durch Tüpfeln der Auslauf geprüft. Ist er rein gelb, so wird nach Zugabe von 10 Teilen Kieselgur abgesaugt, ist er noch braun, so muß erneut Kohle zugegeben werden. Der Rückstand wird mit wenig Wasser gewaschen.

Bereits während der Klärung wird die Temperatur des Ansatzes abgesenkt, was erforderlichenfalls bei der nun folgenden Fällung fortgesetzt wird, bis ca. 15°C erreicht sind. Zur Fällung des bis-Diazoniumsalzes läßt man zu der leicht bräunlichen Diazoniumsalzlösung 450 Teile 33 %ige Zinkchloridlösung zutropfen. Hierbei scheidet sich das 3,3'-Dipropoxybiphenyl-4,4'-bis(diazonium)-tetrachlorozinkat in schönen gelben Kristallen ab. Durch Zugabe von 250 Teilen Natriumchlorid wird die Fällung vervollständigt. Dann wird das bis-Diazoniumsalz abgesaugt und durch Zentrifugieren vom größten Teil der anhaftenden Mutterlauge befreit. Der Restfeuchtgehalt beträgt noch ca. 8 %.

b) Das erhaltene feuchte bis-Diazoniumsalz kann nach der Arbeitsweise von Beispiel 2 a) oder 3 a) in ein haltbares Diazoniumsalzpräparat überführt werden, das in analoger Weise, wie in Beispiel 2 b) oder 3 b) beschrieben, zur Erzeugung von wasserunlöslichen Azofarbstoffen auf Cellulosefasern dienen kann.

Beispiel 5

373 Teile 4,4'-Diamino-3,3'-diisopropoxy-biphenyl-bis-hydrochlorid werden in eine Vorlage aus 700 Teilen Wasser und 465 Teilen 32 %iger Salzsäure eingetragen. Mittels eines Homogenisators (beispielsweise eines (R)Ultraturrax-Geräts) wird eine feine Verteilung erreicht. Durch Außenkühlung wird auf 0°C abgekühlt. Dann werden 370 Teile einer 40 %igen wäßrigen Natriumnitritlösung so zugetropft, daß die Temperatur nicht über 6°C ansteigt und Nitrit zunächst nicht oder nur schwach im Überschuß ist. Nach beendeter Diazotierung soll jedoch ein kräftiger Nitritüberschuß vorhanden sein.

Nun gibt man 10 Teile Klärkohle zu und prüft nach einigen Minuten, ob der Auslauf einer Tüpfelprobe rein gelb ist. Zutreffendenfalls wird über eine mit 10 Teilen Kieselgur beschickte Nutsche abgesaugt, andernfalls muß erneut Kohle zugegeben werden. Der Filterrückstand wird mit wenig Wasser gewaschen.

Zum Filtrat und Waschwasser werden innerhalb 15-30 Minuten stetig, am vorteilhaftesten mittels einer Dosiereinrichtung, 290 Teile Natriumtetrafluoborat gegeben. Das 3,3'-Diisopropoxy-biphenyl-bis(diazoniumtetrafluoborat) fällt in feinen hellgelben Kristallen aus. Diese werden nach einigem Nachrühren abgesaugt und durch Zentrifugieren von überschüssiger Mutterlauge befreit.

Zur Erzeugung eines Färbepräparats wird das so isolierte bis-Diazoniumsalz mit derselben Menge Natriumsulfat vermischt und sodann im Warmluftstrom bei 40°C getrocknet.

Beispiel 6

328 Teile 4,4'-Diamino-3,3'-bis(1-methyl-propoxy)-biphenyl werden, wie in Beispiel 1 beschrieben, in eine Diazoniumsalzlösung überführt.

Zur Fällung des bis-Diazoniumsalzes läßt man zum Filtrat und Waschwasser 220 Teile einer 65 %igen Zinkchloridlösung zutropfen. Das 3,3'-bis(1-Methyl-propoxy)-biphenyl-4,4'-bis(diazonium)tetrachlorozinkat scheidet sich in schönen gelben Kristallen ab. Diese werden nach einigem Nachrühren bei ca. 5°C abgesaugt und durch Zentrifugieren vom größten Teil der anhaftenden Mutterlauge befreit.

Das so erhaltene feuchte Diazoniumsalz wird sodann, wie in Beispiel 3 a) angegeben, in ein Färbepräparat überführt.

**Ansprüche**

1. bis-Diazoniumsalze der allgemeinen Formel

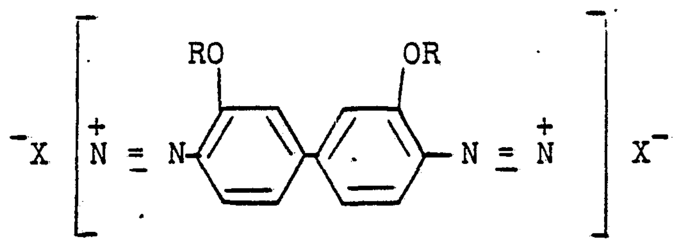

in welcher R einen geradkettigen oder verzweigten Alkyl- oder Alkoxyalkylrest von insgesamt 3-5 Kohlenstoffatomen bedeutet, und $X^-$ den Rest einer starken Säure oder eines Zinkchloriddoppelsalzes darstellt.

2. bis-Diazoniumsalze der in Anspruch 1 genannten allgemeinen Formel, in welcher $X^-$ $Cl^-$, $HSO_3^-$,

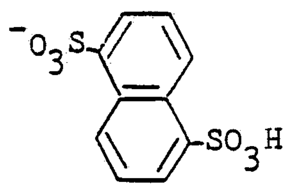

,

$BF_4^-$ oder $\left[\frac{ZnCl_4}{2}\right]^-$ bedeutet.

3. bis-Diazoniumsalze der in Anspruch 1 genannten allgemeinen Formel, in welcher R eine Propyl-, Butyl-, 2-Methoxy-ethoxy-oder 2-Ethoxy-ethoxy-Gruppe bedeutet.

4. Verfahren zur Herstellung von bis-Diazoniumsalzen der allgemeinen Formel

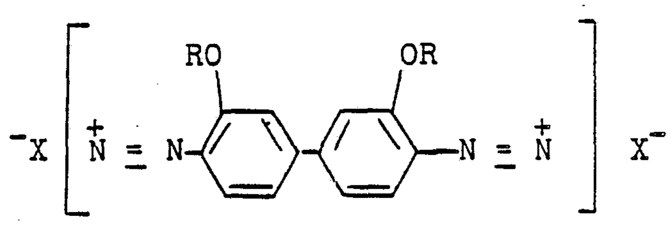

in welcher R einen geradkettigen oder verzweigten Alkyl- oder Alkoxyalkylrest von insgesamt 3-5 Kohlenstoffatomen bedeutet, und $X^-$ den Rest einer starken Säure oder eines Zinkchloriddoppelsalzes darstellt, dadurch gekennzeichnet, daß man ein 4,4'-Diamino-3,3'-dialkoxy-biphenyl der Formel (2)

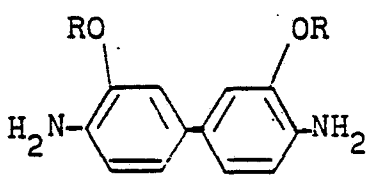

(2)

in welcher R die vorstehend genannten Bedeutungen hat, in einer wäßrigen, starken, nichtoxydierenden anorganischen oder organischen Säure bei Temperaturen von etwa -10°C bis etwa +40°C mit einem Alkalimetallnitrit diazotiert und die gebildeten bis-Diazoniumsalze durch Zugeben eines Alkalimetallchlorids, -hydrogensulfats, -monosulfonats der 1,5-Naphthalindisulfonsäure, -tetrafluoborats oder von Zinkchlorid abscheidet.

5. Verfahren nach Anspruch 4, dadurch gekennzeichnet, daß man die Diazotierung in wäßriger Salzsäure, Schwefelsäure, Phosphorsäure oder Monochloressigsäure bei 0 bis 30 °C durchführt.

6. Verwendung der bis-Diazoniumsalze gemäß Anspruch 1 zum Färben oder Bedrucken von Fasermaterialien aus nativer oder regenerierter Cellulose nach der Eisfarbentechnik.

7. Verwendung der bis-Diazoniumsalze gemäß Anspruch 1 zur Herstellung von Lichtpausen nach den Methoden der Reprografie.